(19) Europäisches Patentamt
European Patent Office
Office européen des brevets

(11) **EP 2 560 638 B1**

(12) **EUROPEAN PATENT SPECIFICATION**

(45) Date of publication and mention
of the grant of the patent:
**11.03.2015  Bulletin 2015/11**

(51) Int Cl.:
***A61K 31/416*** *(2006.01)*      ***A61P 35/00*** *(2006.01)*

(21) Application number: **11772508.5**

(22) Date of filing: **19.04.2011**

(86) International application number:
**PCT/US2011/032938**

(87) International publication number:
**WO 2011/133480 (27.10.2011 Gazette 2011/43)**

(54) **METHOD OF TREATING GASTRIC CANCER**

VERBINDUNG ZUR VERWENDUNG IN DER BEHANDLUNG VON MAGENKREBS

COMPOSÈ POUR l'UTILISATION DANS LE TRAITEMENT D'UN CANCER GASTRIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.04.2010  US 325586 P**

(43) Date of publication of application:
**27.02.2013  Bulletin 2013/09**

(73) Proprietor: **Niiki Pharma Inc.
Hoboken, New Jersey 07030 (US)**

(72) Inventor: **SHESHBARADARAN, Hooshmand
Hoboken, New Jersey 07030 (US)**

(74) Representative: **Vossius & Partner
Siebertstrasse 3
81675 München (DE)**

(56) References cited:
**WO-A1-2008/154553      US-A- 4 843 069
US-A1- 2010 094 019**

- **PETRA HEFFETER ET AL: "Intracellular protein binding patterns of the anticancer ruthenium drugs KP1019 and KP1339", JBIC JOURNAL OF BIOLOGICAL INORGANIC CHEMISTRY, SPRINGER, BERLIN, DE, vol. 15, no. 5, 11 March 2010 (2010-03-11) , pages 737-748, XP019854157, ISSN: 1432-1327**
- **CHRISTIAN G. HARTINGER ET AL.: 'From bench to bedside-preclinical and early clinical development of the anticancer agent indazolium trans-[tetra- chlorobis(IH-indazole)ruthenate(III)] (KP1019 or FFC14A)' JOURNAL OF INORGANIC BIOCHEMISTRY vol. 100, no. 5-6, 2006, pages 891 - 904, XP027900388**
- **S. KAPITZA ET AL.: 'Heterocyclic complexes of ruthenium(III) induce apoptosis in colorectal carcinoma cells.' JOURNAL OF CANCER RESEARCH AND CLINICAL ONCOLOGY vol. 131, no. 2, 2005, pages 101 - 110, XP019345379**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Cross-Reference to Related U.S. Applications

This application claims the benefit of U.S. Provisional Application No. 61/325,586 filed on April 19, 2010.

Field of the Invention

The present invention generally relates to pharmaceutical compositions for treating cancer, and particularly to a pharmaceutical composition having a ruthenium(III) complex.

Background of the Invention

Gastric cancer is one of the most deadly forms of cancer. Treatment option for gastric cancer has been limited. Surgery and radiation therapy can be used for early-stage gastric cancer, but not very effective for advanced or recurrent gastric cancer. Traditional chemotherapeutic agents such as 5-fluorouracil and cisplatin have shown very limited effect often causing serious side effects. Thus, there is a significant unmet need for new agents and methods for treating gastric cancer.

Summary of the Invention

The present invention provides methods of treating gastric cancer. In one aspect, the present invention provides a method of treating, preventing or delaying the onset of, gastric cancer, comprising administering to a patient having gastric cancer a therapeutically or prophylatically effective amount of a pharmaceutically acceptable salt of trans-[tetrachlorobis(1H-indazole)ruthenate(III)] (e.g., sodium trans-[tetrachlorobis(1H-indazole)ruthenate(III)] or potassium trans-[tetrachlorobis(1H-indazole)ruthenate(III)]).

In accordance with another aspect, a method of treating, preventing or delaying the onset of, a refractory gastric cancer is provided comprising administering a therapeutically or prophylatically effective amount of an alkali metal salt of trans-[tetrachlorobis(1H-indazole)ruthenate(III)] (e.g., sodium trans-[tetrachlorobis(1H-indazole)ruthenate(III)] or potassium trans-[tetrachlorobis(1H-indazole)ruthenate(III)]) to a patient having refractory gastric cancer.

Use of an alkali metal salt of trans-[tetrachlorobis(1H-indazole)ruthenate(III)] (e.g., sodium trans-[tetrachlorobis(1H-indazole)ruthenate(III)] or potassium trans-[tetrachlorobis(1H-indazole)ruthenate(III)]) for the manufacture of a medicament for use in the methods of the present invention is also provided.

The foregoing and other advantages and features of the invention, and the manner in which the same are accomplished, will become more readily apparent upon consideration of the following detailed description of the invention taken in conjunction with the accompanying examples, which illustrate preferred and exemplary embodiments.

Brief Description of the Drawings

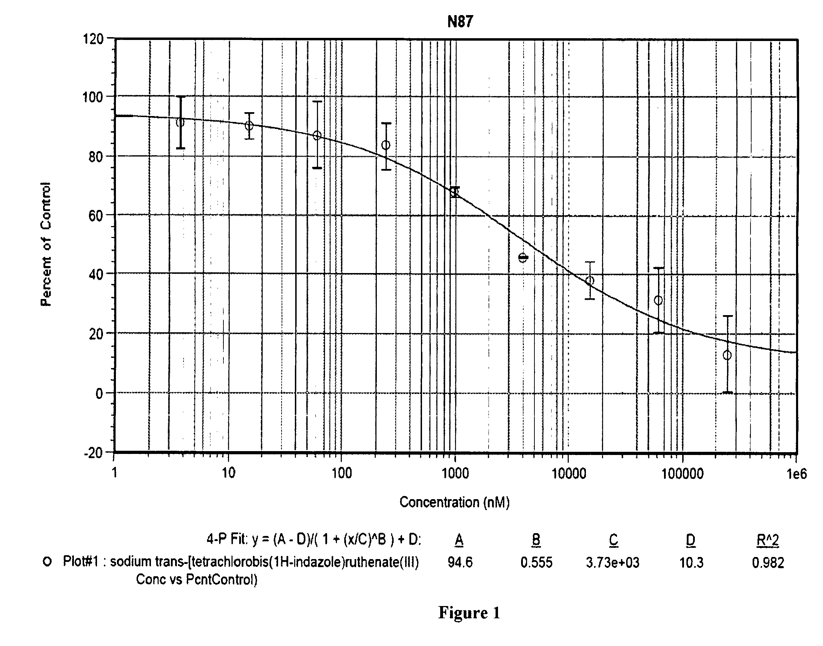

Figure 1 is a graph showing the dose-dependent growth inhibition by sodium trans-[tetrachlorobis(1H-indazole)ruthenate(III)] in an MTT assay in gastric cancer cell line NCI-N87. X axis is drug concentration in nM and Y axis is percentage of control in absorbance.

Detailed Description of the Invention

The present invention is at least in part based on the discovery that the compound sodium trans-[tetrachlorobis(1H-indazole)ruthenate(III)] is particularly effective in treating gastric cancer. Accordingly, in accordance with a first aspect of the present invention, a method is provided for treating gastric cancer. The method comprises treating a gastric cancer patient in need of treatment with a therapeutically effective amount of trans-[tetrachlorobis(1H-indazole)ruthenate(III)] or a pharmaceutically acceptable salt thereof. Examples of such a salt include an indazolium salt or alkali metal salt of trans-[tetrachlorobis(1H-indazole)ruthenate(III)] (e.g., sodium trans-[tetrachlorobis(1H-indazole)ruthenate(III)] or potassium trans-[tetrachlorobis(1H-indazole)ruthenate(III)]). That is, the present invention is directed to the use of an effective amount of a trans-[tetrachlorobis(1H-indaaole)ruthenate(III)] or a pharmaceutically acceptable salt thereof (e.g., indazolium or alkali metal salt) for the manufacture of medicaments for treating a gastric cancer in patients identified or diagnosed as having a gastric cancer.

The compound of the present invention can be useful in various gastric malignancies including, but not limited to, gastric adenocarcinoma, MALT lymphoma (MALToma), stromal tumors, and gastrointestinal stromal tumor (GIST).

In the various embodiments of this aspect of the present invention, the treatment method optionally also comprises a step of diagnosing or identifying a patient as having gastric tumor. The identified patient is then treated with or administered with a therapeutically effective amount of trans-[tetrachlorobis(1H-indazole)ruthenate(III)] or a pharmaceutically acceptable salt thereof, preferably an alkali metal salt, e.g., sodium trans-[tetrachlorobis(1H-indazole)ruthenate(III)]. Various gastric cancers can be diagnosed in any conventional diagnostic methods known in the art including CT scan, endoscopy, barium roentgenogram, biopsy, etc.

In addition, another aspect of the present invention provides a compound for treating refractory gastric cancer comprising treating a patient identified as having refractory gastric cancer with a therapeutically effective amount of trans-[tetrachlorobis(1H-indazole)ruthenate(III)] or a pharmaceutically acceptable salt thereof, preferably an alkali metal salt thereof (e.g., sodium trans-[tetrachlorobis(1H-indazole)ruthenate(III)] or potassium trans-[tetrachlorobis(1H-indazole)ruthenate(III)]).

The term "refractory gastric cancer" as used herein refers to a gastric cancer that either fails to respond favorably to an anti-neoplastic treatment that does not include trans-[tetrachlorobis(1H-indazole)ruthenate(III)] or a pharmaceutically acceptable salt thereof, or alternatively, recurs or relapses after responding favorably to an antineoplastic treatment that does not include trans-[tetrachlorobis(1H-indazole)ruthenate(III)] or a pharmaceutically acceptable salt thereof. Accordingly, "a gastric cancer refractory to a treatment" as used herein means a gastric cancer that fails to respond favorably to, or resistant to, the treatment, or alternatively, recurs or relapses after responding favorably to the treatment.

To detect a refractory gastric cancer, patients undergoing initial treatment can be carefully monitored for signs of resistance, non-responsiveness or recurring gastric cancer. This can be accomplished by monitoring the patient's cancer's response to the initial treatment. The response, lack of response, or relapse of the cancer to the initial treatment can be determined by any suitable method practiced in the art. For example, this can be accomplished by the assessment of tumor size and number. An increase in tumor size or, alternatively, tumor number, indicates that the tumor is not responding to the chemotherapy, or that a relapse has occurred. The determination can be done according to the "RECIST" criteria as described in detail in Therasse et al, J. Natl. Cancer Inst. 92:205-216 (2000).

In accordance with yet another aspect of the present invention, a method is provided for preventing or delaying the onset of gastric cancer, or preventing or delaying the recurrence of gastric cancer, which comprises treating a patient in need of the prevention or delay with a prophylatically effective amount of trans-[tetrachlorobis(1H-indazole)ruthenate(III)] or a pharmaceutically acceptable salt thereof, preferably an alkali metal salt of trans-[tetrachlorobis(1H-indazole)ruthenate(III)] (e.g., sodium trans-[tetrachlorobis(1H-indazole)ruthenate(III)] or potassium trans-[tetrachlorobis(1H-indazole)ruthenate(III)]).

For purposes of preventing or delaying the recurrence of gastric cancer, patients with gastric cancer who have been treated and are in remission or in a stable or progression free state may be treated with a prophylatically effective amount of an alkali metal salt of trans-[tetrachlorobis(1H-indazole)ruthenate(III)] (e.g., sodium trans-[tetrachlorobis(1H-indazole)ruthenate(III)] or potassium trans-[tetrachlorobis(1H-indazole)ruthenate(III)]) to effectively prevent or delay the recurrence or relapse of gastric cancer.

As used herein, the phrase "treating ... with ..." or a paraphrase thereof means administering a compound to the patient or causing the formation of a compound inside the body of the patient.

In accordance with the present invention, gastric cancer can be treated with a therapeutically effective amount of trans-[tetrachlorobis(1H-indazole)ruthenate(III)] or a pharmaceutically acceptable salt thereof, preferably an alkali metal salt of trans-[tetrachlorobis(1H-indazole)ruthenate(III)], alone as a single agent, or alternatively in combination with one or more other anti-cancer agents.

Alkali metal salts of trans-[tetrachlorobis(1H-indazole)ruthenate(III)] can be made in any methods known in the art. For example, PCT Publication No. WO/2008/154553 discloses an efficient method of making sodium trans-[tetrachlorobis(1H-indazole)ruthenate(III)].

The pharmaceutical compounds such as sodium trans-[tetrachlorobis(1H-indazole)ruthenate(III)] can be administered through intravenous injection or any other suitable means at an amount of from 0.1 mg to 1000 mg per kg of body weight of the patient based on total body weight. The active ingredients may be administered at once, or may be divided into a number of smaller doses to be administered at predetermined intervals of time, e.g., once daily or once every two days. It should be understood that the dosage ranges set forth above are exemplary only and are not intended to limit the scope of this invention. The therapeutically effective amount of the active compound can vary with factors including, but not limited to, the activity of the compound used, stability of the active compound in the patient's body, the severity of the conditions to be alleviated, the total weight of the patient treated, the route of administration, the ease of absorption, distribution, and excretion of the active compound by the body, the age and sensitivity of the patient to be treated, and the like, as will be apparent to a skilled artisan. The amount of administration can be adjusted as the various factors change over time.

In accordance with the present invention, it is provided a use of trans-[tetrachlorobis(1H-indazole)ruthenate(III)] or a pharmaceutically acceptable salt thereof, such as an alkali metal salt of trans-[tetrachlorobis(1H-indazole)ruthenate(III)] (e.g., sodium trans-[tetrachlorobis(1H-indazole)ruthenate(III)] or potassium trans-[tetrachlorobis(1H-indazole)ruthenate(III)]), for the manufacture of a medicament useful for treating gastric cancer. The medicament can be, e.g., in an injectable form, e.g., suitable for intravenous, intradermal, or intramuscular administration. Injectable forms are generally known in the art, e.g., in buffered solution or suspension.

In accordance with another aspect of the present invention, a pharmaceutical kit is provided comprising in a container a unit dosage form of trans-[tetrachlorobis(1H-indazole)ruthenate(III)] or a pharmaceutically acceptable salt thereof, such as an alkali metal salt of trans-[tetrachlorobis(1H-indazole)ruthenate(III)] (e.g., sodium trans-[tetrachlorobis(1H-indazole)ruthenate(III)] or potassium trans-[tetrachlorobis(1H-indazole)ruthenate(III)]), and optionally instructions for using the kit in the methods in accordance with the present invention, e.g., treating, preventing or delaying the onset of gastric cancer, or preventing or delaying the recurrence of gastric cancer, or treating refractory gastric cancer. As will be apparent to a skilled artisan, the amount of a therapeutic compound in the unit dosage form is determined by the dosage to be used on a patient in the methods of the present invention. In the kit, trans-[tetrachlorobis(1H-indazole)ruthenate(III)] or a pharmaceutically acceptable salt thereof such as an alkali metal salt of trans-[tetrachlorobis(1H-indazole)ruthenate(III)] (e.g., sodium trans-[tetrachlorobis(1H-indazole)ruthenate(III)] or potassium trans-[tetrachlorobis(1H-indazole)ruthenate(III)]) can be in lyophilized form in an amount of, e.g., 25 mg, in an ampoule. In the clinic, the lyophilized form can be dissolved and administered to a patient in need of the treatment in accordance with the present invention.

EXAMPLE 1

To test the activities of sodium trans-[tetrachlorobis(1H-indazole)ruthenate(III)], ATCC's MTT Cell Proliferation Assay® was performed using human gastric cancer cell line NCI-N87 (differentiated carcinoma). Stock cultures were allowed to grow to 70-80% confluence for this study. The anti-proliferative activity of sodium trans-[tetrachlorobis(1H-indazole)ruthenate(III)], against the indicated cell line was evaluated *in vitro* using the the ATCC's MTT Cell Proliferation Assay (Catalog No. 30-1010K). NCI-N87 was grown using RPMI1640 (Cell Gro 10-040-CV), with 1% of 1M HEPES, 1% sodium pyruvate, 1% of 45% glucose solution, 10% of heat-inactivated FBS and 1% of pen/strep/glutamine. NCI-N87 cell plates were seeded with 20E+03 cells/well and treated with sodium trans-[tetrachlorobis(1H-indazole)ruthenate(III)] at 1,000 $\mu$M, or a series of 4x dilutions thereof (250 $\mu$M, 62.5 $\mu$M, etc.). 100$\mu$l of medium was removed from each well at 72 hours post-treatment and 10$\mu$l MTT reagent was added to each well. The plates were incubated plate at 37°C for 4 hours and then 100$\mu$l of detergent was added. The plates were left overnight at room temperature in the dark and was read on a plate reader using SoftMax® Pro (version 5.2, Molecular Devices).

The absorbance data was analyzed as follows: Absorbance values were converted to Percent of Control and plotted against test agent concentrations for IC$_{50}$ calculations using SoftMax® Pro (version 5.2, Molecular Devices). The plate blank signal average was subtracted from all wells prior to calculating the Percent of Control. Percent of Control values were calculated by dividing the absorbance values for each test well by the No Drug Control average (column 11 values; cells + vehicle control) and multiplying by 100. Plots of Compound Concentration versus Percent of Control were analyzed using the 4-parameter equation to obtain IC$_{50}$ values and other parameters that describe the sigmoidal dose response curve.

The IC$_{50}$ value for the test agents was estimated by curve-fitting the data using the following four parameter-logistic equation:

$$ Y = \frac{Top - Bottom}{1 + \left( X \middle/ IC_{50} \right)^{n}} + Bottom $$

wherein "Top" is the maximal % of control absorbance (100%) (Value "A" in Figure 1), "Bottom" is the minimal % of control absorbance at the highest agent concentration (down to zero) (Value "D" in Figure 1), Y is the Percent of Control absorbance, X is the test agent Concentration, IC$_{50}$ is the concentration of agent that inhibits cell growth by 50% compared to the control cells (Value "C" in Figure 1), n is the slope of the curve (Value "B" in Figure 1). The IC$_{50}$ of sodium trans-[tetrachlorobis(1H-indazole)ruthenate(III)] in NCI-N87 cell line was 3.73 $\mu$M.

All publications and patent applications mentioned in the specification are indicative of the level of those skilled in the art to which this invention pertains. The mere mentioning of the publications and patent applications does not necessarily constitute an admission that they are prior art to the instant application.

Although the foregoing invention has been described in some detail by way of illustration and example for purposes of clarity of understanding, it will be apparent that certain changes and modifications may be practiced within the scope of the appended claims.

**Claims**

1. Use of a pharmaceutically acceptable salt of trans-[tetrachlorobis(1H-indazole)ruthenate(III)] for the manufacture of a medicament for treating, preventing, or delaying the onset of, gastric cancer.

2. The use of Claim 1, wherein said salt is an alkali metal salt of trans-[tetrachlorobis(1H-indazole)ruthenate(III)].

3. The use of Claim 1, wherein said pharmaceutically acceptable salt of trans-[tetrachlorobis(1H-indazole)ruthenate(III)] is sodium trans-[tetrachlorobis(1H-indazole)ruthenate(III)].

4. A pharmaceutically acceptable salt of trans-[tetrachlorobis(1H-indazole)ruthenate(III)] for use in treating, preventing, or delaying the onset of, gastric cancer.

5. The pharmaceutically acceptable salt of trans-[tetrachlorobis(1H-indazole)ruthenate(III)] for use according to Claim 4, wherein said salt is an alkali metal salt of trans-[tetrachlorobis(1H-indazole)ruthenate(III)].

**6.** The pharmaceutically acceptable salt of trans-[tetrachlorobis(1H-indazole)ruthenate(III)] for use according to Claim 4, wherein said pharmaceutically acceptable salt of trans-[tetrachlorobis(1H-indazole)ruthenate(III)] is sodium trans-[tetrachlorobis(1H-indazole)ruthenate(III)].

**Patentansprüche**

**1.** Verwendung eines pharmazeutisch verträglichen Salzes von trans-[tetrachlorobis(1H-indazol)ruthenat(III)] zur Herstellung eines Medikaments zur Behandlung, Vorbeugung oder Verzögerung des Ausbruchs von Magenkrebs.

**2.** Verwendung nach Anspruch 1, wobei das Salz ein Alkalimetallsalz von trans-[tetrachlorobis(1H-indazol)ruthenat(III)] ist.

**3.** Verwendung nach Anspruch 1, wobei das pharmazeutisch verträgliche Salz von trans-[tetrachlorobis(1H-indazol)ruthenat(III)] Natrium-trans-[tetrachlorobis(1H-indazol)ruthenat(III)] ist.

**4.** Pharmazeutisch verträgliches Salz von trans-[tetrachlorobis(1H-indazol)ruthenat(III)] zur Verwendung in der Behandlung, Vorbeugung oder Verzögerung des Ausbruchs von Magenkrebs.

**5.** Pharmazeutisch verträgliches Salz von trans-[tetrachlorobis(1H-indazol)ruthenat(III)] zur Verwendung nach Anspruch 4, wobei das Salz ein Alkalimetallsalz von trans-[tetrachlorobis(1H-indazol)ruthenat(III)] ist.

**6.** Pharmazeutisch verträgliches Salz von trans-[tetrachlorobis(1H-indazol)ruthenat(III)] zur Verwendung nach Anspruch 4, wobei das pharmazeutisch verträgliche Salz von trans-[tetrachtorobis(1H-indazol)ruthenat(III)] Natrium-trans-[tetrachlorobis(1H-indazol)ruthenat(III)] ist.

**Revendications**

**1.** Utilisation d'un sel pharmaceutiquement acceptable de trans-[tétrachlorobis(1H-indazole)ruthénate(III)] pour la fabrication d'un médicament destiné au traitement, à la prévention ou au retardement de l'apparition d'un cancer gastrique.

**2.** Utilisation selon la revendication 1, dans laquelle ledit sel est un sel de métal alcalin de trans-[tétrachlorobis(1H-indazole)ruthénate(III)].

**3.** Utilisation selon la revendication 1, dans laquelle ledit sel pharmaceutiquement acceptable de trans-[tétrachlorobis(1H-indazole)ruthénate(III)] est le trans-[tétrachlorobis(1H-indazole)ruthénate(III)] de sodium.

**4.** Sel pharmaceutiquement acceptable de trans-[tétrachlorobis(1H-indazole)ruthénate(III)] pour une utilisation dans le traitement, la prévention, ou le retardement de l'apparition d'un cancer gastrique.

**5.** Sel pharmaceutiquement acceptable de trans-[tétrachlorobis(1H-indazole)ruthénate(III)] pour une utilisation selon la revendication 4, dans lequel ledit sel est un sel de métal alcalin de trans-[tétrachlorobis(1H-indazole)ruthénate(III)].

**6.** Sel pharmaceutiquement acceptable de trans-[tétrachlorobis(1H-indazole)ruthénate(III)] pour une utilisation selon la revendication 4, dans lequel ledit sel pharmaceutiquement acceptable de trans-[tétrachlorobis(1H-indazole)ruthénate(III)] est le trans-[tétrachlorobis(1H-indazole)ruthénate(III)] de sodium.

**N87**

4-P Fit: y = (A - D)/( 1 + (x/C)^B ) + D:

| | A | B | C | D | R^2 |
|---|---|---|---|---|---|
| O Plot#1 : sodium trans-[tetrachlorobis(1H-indazole)ruthenate(III) Conc vs PcntControl) | 94.6 | 0.555 | 3.73e+03 | 10.3 | 0.982 |

**Figure 1**

## EP 2 560 638 B1

### REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61325586 A

- WO 2008154553 A

**Non-patent literature cited in the description**

- **THERASSE et al.** *J. Natl. Cancer Inst.,* 2000, vol. 92, 205-216